# EUROPEAN PATENT APPLICATION

(11) **EP 4 813 314 A1**
(43) Date of publication of application: **30.09.2026**
(21) Application number: 25166572.5
(22) Date of filing: 27.03.2025
(51) Int. Cl.: A61B 8/08, A61B 8/00

(54) **ASSISTING AN ULTRASONIC EXAMINATION OF AN OBJECT**

(71) Applicant: Siemens Healthineers AG, 91301 Forchheim (DE)
(72) Inventor: Alzaga, Amilcar, 96185 Schönbrun im Steigerwald (DE); Kellnberger, Stephan, 85604 Zorneding (DE)
(74) Representative: Siemens Healthineers Patent Attorneys

(57) **Abstract**

Method for assisting a placement of an ultrasonic transducer (1) on an object (2) to monitor a medical device (3), wherein a target region (9) inside the object (2) and predetermined imaging data (10) are received by a data processing system (5), and wherein a target device path (11) for the medical device (3) to the target region (9) and an acoustic window for the target device path (11) are determined by the data processing system (5) depending on the predetermined imaging data (10). The method further comprises determining a target transducer position (15a) depending on the acoustic window, receiving a current transducer position (8a) and generating a transducer assisting feedback (4) for assisting a user (7). The method furthermore comprises determining a target transducer orientation (15b) depending on the acoustic window, receiving a current transducer orientation (8b) and generating the transducer assisting feedback (4) for assisting the user (7).

## Description

The invention relates to a method for assisting a placement of an ultrasonic transducer on an object to monitor a medical device, wherein a target region inside the object and predetermined imaging data of the object or of a part of the object are received. Additionally, the invention relates to a data processing system, which is configured to carry out the method, a corresponding user assistance system and a computer program product.

Monitoring a medical device, for example an ablation needle, which shall be injected into an object, for example into a patient's body, during a percutaneous intervention may be crucial for a success of the percutaneous intervention as well as for reducing a risk of delay of the intervention or a risk of injury of the patient.

Currently, percutaneous interventions using medical devices, such as needles, may be performed under monitoring by a computed tomography (CT) system. In this case, a user may progress the medical device under CT monitoring in a step-and-shoot manner. Specifically, the user may position the medical device on a surface of the object, for example a skin of the patient, and may iteratively progress the medical device while starting a CT scan to check a current position of the medical device. This procedure may require multiple CT scans during which the user may have to leave an interventional suite. Furthermore, the multiple CT scans lead to a significant X-ray dose applied to the patient.

Ultrasonic monitoring for percutaneous interventions may offer numerous benefits, including real-time visualization, reduced radiation exposure, and an ability to perform the ultrasonic monitoring at a bedside. However, the ultrasonic monitoring may come with challenges, such as a need for enhanced spatial orientation skills and proper handling and interpretation of image information and implementation in real time actions. These factors contribute to making ultrasonic monitoring highly user-dependent, as a successful outcome may be closely related to the user's expertise and experience.

A significant challenge in ultrasonic monitoring for percutaneous procedures, such as ablations, may be achieving proper spatial orientation and determining a correct placement of an ultrasonic transducer to accurately monitor the medical device to a target region.

The user may have to reach the target region with the medical device, where a desired treatment is possible. The position will require a target device path for the medical device, which does not cross any structure that should be preserved intact. This may for example be a vessel, the bowel or other adjacent organs of the patient. In addition to this, ultrasonic monitoring may only be possible, if the target region and the target device path for the medical device are both visible on imaging data generated by an ultrasonic transducer. A limitation of ultrasonic imaging may be that an ultrasonic wave may not be able to penetrate specific structures of the patient. Thus, the specific structures, for example bones of gas, may mask or cover other structures, which are positioned in an acoustic shadow of the specific structures for a field of view of the ultrasonic transducer at a current transducer position. It may require significant experience and spatial orientation for the user to find a current transducer position, which may enable a generation of ultrasonic images, which cover the target region as well as the target device path for the medical device.

The publication R. Göbl et al.: "Acoustic window planning for ultrasound acquisition.", Int. J. Comput. Assist. Radiol. Surg. 2(6):993-1001 proposes an approach for the planning of acquisition trajectories for ultrasonic transducers, which is based on information about the target region as well as tomographic imaging data, such as CT data. The approach includes determining appropriate acoustic windows for the target region.

It is an objective of the invention to assist a user at placing an ultrasonic transducer on an object in order to monitor a medical device, in particular to monitor a target region and/or a target device path for the medical device.

This objective is achieved by the subject matter of the independent claim. Further implementations and preferred embodiments are subject matter of the dependent claims.

The invention is based on the idea to determine a target device path for the medical device to the target region and an acoustic window for the target device path based on predetermined imaging data and to support precise placement of the ultrasonic transducer to monitor the target device path.

According to an aspect of the intervention, a method for assisting a placement of an ultrasonic transducer on an object to monitor a medical device is provided. A target region inside the object and predetermined imaging data of the object or of a part of the object are received by a data processing system. A target device path for the medical device, in particular from a surface of the object, to the target region and an acoustic window for the target device path are determined by the data processing system depending on the predetermined imaging data. The method further comprises determining a target transducer position for the ultrasonic transducer by the data processing system depending on the acoustic window, receiving a current transducer position of the ultrasonic transducer from a transducer monitoring system by the data processing system and generating a transducer assisting feedback for assisting a user in placing the ultrasonic transducer on the object depending on the target transducer position and the current transducer position by the data processing system. Additionally or alternatively the method comprises determining a target transducer orientation for the ultrasonic transducer by the data processing system depending on the acoustic window, receiving a current transducer orientation of the ultrasonic transducer from the transducer monitoring system by the data processing system and generating the transducer assisting feedback for assisting the user in placing the ultrasonic transducer on the object depending on the target transducer orientation and the current transducer orientation by the data processing system.

Within the present disclosure the terms "ultrasound" and "ultrasonic" are used with the same meaning.

Unless stated otherwise, all steps of the method may be performed by the data processing system, which comprises at least one data processing device. In particular, the at least one data processing device is configured or adapted to perform the steps of the method. For this purpose, the at least one data processing device may for example store a computer program comprising instructions which, when executed by the at least one data processing device, cause the at least one data processing device to execute the method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

The predetermined imaging data may inter alia represent the target region as part of the object. In particular, the target region may have been identified by the user manually or semiautomatically using the data processing system based on the predetermined imaging data or based on further imaging data representing the object the target region may have been identified automatically by the data processing system based on the predetermined imaging data or based on the further imaging data representing the object. The target region may be a part of the object, which shall be treated by the medical device or from which a specimen shall be taken by the medical device, for example. The target region may for example be an organ of the patient or a part of an organ, such as a pathological change of an organ or a tumor or a lesion. It is emphasized that the method according to the invention does not include the identification of the target region or any other diagnostic step. Furthermore, it is emphasized that the method according to the invention does not include carrying out any steps to insert the medical device into the object or to move the medical device in the object or to manipulate the object by the medical device or any other surgical steps.

The predetermined imaging data may represent the object or the part of the object without the medical device. In other words, the medical device may not be represented by the predetermined imaging data. The predetermined imaging data may have been generated prior to an execution of the method. In other words, the predetermined imaging data may be pre-interventional imaging data. The predetermined imaging data may be generated by an imaging system, such as a CT-system or an X-ray based fluoroscopy system or an MRI-system.

In particular, the predetermined imaging data may be two-dimensional imaging data. In this case, the target region is for example a point or a two-dimensional region. Preferably, the predetermined imaging data may be three-dimensional imaging data. In this case, the target region is for example a point or a three-dimensional region.

The target device path may be determined considering regions inside the object, which shall not be affected, touched or penetrated by the medical device. In such a case, the target device path may avoid these regions and the data processing system may find an alternative target device path.

The determination of the target device path may consider a possibility to monitor the target device path via the acoustic window by the ultrasonic transducer. This consideration may be depending on a position of a specific structure, which an ultrasonic wave may not be able to penetrate. In particular, the determination of the target device path may be determined such that generating ultrasonic imaging data, which represent a complete target device path, by the ultrasonic transducer is enabled. The complete target device path may start at the surface of the object, for example the skin of the patient, and may end at the target region, for example the part of the organ of the patient, or at a boundary of the target region.

For example, the medical device may be or comprise a needle, for example a stiff needle. For example, the medical device may be or comprise an ablation needle or a biopsy needle. For example, the medical device may be or comprise a vessel catheter and/or a guide wire and/or a stent or another vessel implant.

In particular, the target device path may consist of a straight or a curved line. The target device path may lead via the straight or the curved line starting from the surface of the object to the target region within the object. Alternatively, the target device path may comprise one or more straight lines and/or one or more curved lines, which are connected to each other.

The acoustic window may be a part of the surface of the object, which is suitable for the placement of the ultrasonic transducer in order to enable generating ultrasonic imaging data, which represents the target device path, in particular the complete target device path, by the ultrasonic transducer. The acoustic window may comprise a single area or two or more discontiguous areas, for example a subset of the surface of the object.

The determination of the acoustic window may depend on the target device path, in particular the complete target device path, and the target region, which may be located at an end of the target device path and the specific structure, which an ultrasonic wave may not be able to penetrate. It is also possible that the acoustic window and the target device path are determined jointly based on the predetermined imaging data. The predetermined imaging data may represent necessary information for the determination of the acoustic window.

The determination of the acoustic window and/or the determination of the target device path may comprise a deterministic optimization and/or a deterministic solver, for example a pareto optimization or a pareto solver of a weighted sum optimization or a weighted sum solver.

The target transducer position may be located within the acoustic window, in particular may be located within the single area or the two or more discontiguous areas. The target transducer position may depend on an accessibility of the ultrasonic transducer for example by the user, in particular considering the target device path. In other words, the user may for example have to be able to manually reach a starting position of the target device path with the medical device and simultaneously be able to manually reach the target transducer position with the ultrasonic transducer. The target transducer position may also consider a position of the object, in particular a resting position of the patient. For example, the starting position of the target device path and the target transducer position may be accessible by the user even when the patient is located in a stable position on a patient support or bed.

The user may, in particular, be an operator for the medical intervention, especially a physician or a physician assistant. In particular, it may be a single person, who is being assisted by the assisting method and who is handling the medical device and the ultrasonic transducer. Additionally, a further person may be assisted by the assisting method and for example act as an auditing body or as a support for the single person. During the ultrasonic scan, the user may hold the ultrasonic transducer in one hand and for example the medical device in the other hand, alternatively, the further person may hold one of the two. The ultrasonic transducer may alternatively or additionally comprise a holder and/or a bracket that is clamped to the ultrasonic transducer. The medical device may be supported and/or progressed by the holder and/or the bracket. In such an embodiment the user may be able to hold the ultrasonic transducer and the medical device in one hand.

The transducer monitoring system may be configured to generate a pose signal, which comprises information about the current transducer position and/or the current transducer orientation. The pose signal may be provided to the data processing system in order to generate the transducer assisting feedback. The method may comprise comparing the current transducer position with the target transducer position and may generate a position feedback as part of the transducer assisting feedback. Additionally or alternatively the method may comprise comparing the current transducer orientation with the target transducer orientation and may generate an orientation feedback as part of the transducer assisting feedback.

An intention of the transducer assisting feedback may be to indicate a required or proposed change of the current transducer position and/or of the current transducer position, for example to inform the user to actively initiate the change. An intensity of the transducer assisting feedback and/or an actual implementation of the transducer assisting feedback may depend on a criticality of a deviation of current transducer position from the target transducer position and/or on a criticality of a deviation of the current transducer orientation from the target transducer orientation.

An advantage of the invention is the assistance of the user in order to shorten the duration of the medical intervention. The method provides a solution for finding a combination of the target device path and the acoustic window depending on the predetermined imaging data and assists the user to find an optimal pose for the ultrasonic transducer in order to follow a progress of the medical device. This significantly improves the handling of the monitored intervention for the user and may also increase the success rate of the intervention results. The method may enable users with less experience or expertise to perform a successful intervention as the method assists these users to plan the intervention and to permanently identify a correct transducer pose during the execution of the intervention. Next to that, the method does not require a fixed or permanent installation of equipment to a medical building or apparatus, such as a laser guidance system within an imaging system of a hospital or the like. Furthermore, the method allows to reduce an overall X-ray dose to be applied to the object.

According to several implementations, the determination of the target device path and the acoustic window is carried out jointly, wherein the target device path is determined such that ultrasonic imaging of the medical device along the target device path is possible according to the acoustic window.

That the ultrasonic imaging of the medical device along the target device path is possible may be understood such that an acoustic path from the target transducer position to the target device path, in particular to the complete target device path, is free of structures, which block or absorb the ultrasonic wave completely or to such an extent that ultrasonic imaging of objects lying behind said structures is prevented.

For example, the joint determination of the target device path and the acoustic window may include a mutual or alternating optimization process, which may consider both an optimization of the target device path and the acoustic window simultaneously or alternatingly. The determination may consider first constraints for the target device path and second constraints for the acoustic window at the same time. The determination may depend on user input or preferences, which the user may set prior to the determination. For example, the user may mask or deactivate undesired solutions for the target device path and/or for the acoustic window, in particular undesired solutions may depend on a condition of the patient.

Additionally or alternatively the determination of the target device path and the acoustic window may result in several solutions for both. The user may for example be able to choose a preferred solution of the several solutions.

An advantage of such implementations is that the determination may be able to achieve improved results compared to a sequential approach, when first the target device path is determined and based on that the acoustic window is determined.

According to several further implementations, the target device path and the acoustic window are determined by applying a machine learning model (MLM), in particular a trained machine learning model, to input data, which depends on, for example comprises or consists of, the predetermined imaging data.

In general terms, an MLM, in particular a trained MLM, may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network, GAN.

In particular, by assisting the user using the method according to the invention, a significant contribution to getting an improved solution for the target device path and the acoustic window may be made. Thus, the intervention planning is improved by the MLM.

In some implementation, the MLM is trained in a supervised fashion based on training data, which includes a plurality of training imaging datasets and respective target regions defined in each of the training imaging datasets. The training data also includes respective annotations or ground truths for a respective acoustic window and a respective target device path for each training imaging dataset.

The MLM is applied to one of the training imaging datasets to predict a respective predicted target device path and a respective predicted acoustic window. A predefined loss function is evaluated depending on the predicted target device path and the predicted acoustic window as well as the respective ground truths, in particular depending on respective deviations of the predicted target device path and the predicted acoustic window from the ground truths. The MLM is the adapted or updated depending on a result of the evaluation of the loss function. This is repeated for all training imaging datasets or at least for a subset of the plurality of training imaging datasets until a predefined termination criterion or convergence criterion is met.

According to several further implementations, generating the transducer assisting feedback comprises
- displaying a visualization of the acoustic window by at least one display device; and/or
- displaying a visualization of the target device path by the at least one display device; and/or
- comparing the target transducer position with the current transducer position and/or comparing the target transducer orientation with the current transducer orientation by the data processing system, generating a visualization of a field of view of the ultrasonic transducer for the current transducer position and/or the current transducer orientation depending on a result of the comparison by the data processing system, and displaying the visualization of the field of view by the at least one display device; and/or
- displaying a visualization of a proposed user action to adjust the placement of the ultrasonic transducer to reach the target transducer position starting from the current transducer position and/or to reach the target transducer orientation starting from the current transducer orientation;
- generating a haptic feedback and/or vibrotactile feedback to the user depending on the result of the comparison by the ultrasonic transducer; and/or
- generating a visual feedback to the user via at least one light source of the ultrasonic transducer depending on the result of the comparison; and/or
- generating a visualization of a further field of view of the ultrasonic transducer for the target transducer position and/or the target transducer orientation, and displaying the visualization of the further field of view by the at least one display device.

The at least one display device may for example display an overlaid view including for example the visualization of the field of view and/or the visualization of the target device path and a visualization of the predefined imaging data. The at least one display device may be a mounted or positioned device.

The at least one light source may for example include one or more LEDs, in particular LED indicators, of the ultrasonic transducer.

The field of view of the ultrasonic transducer may comprise device specific features, such as an opening angle of a visible area of the ultrasonic transducer, a width of the visible area and a penetration depth. The visualization of the field of view may comprise for example comprise a color code depending on the result of the comparison. For example, a green background color of the field of view may indicate that the current transducer position and/or the current transducer orientation is identical or similar to the target transducer position and/or the target transducer orientation. An orange background color of the field of view may indicate that the current transducer position and/or the current transducer orientation comprise a first deviation of the target transducer position and/or the target transducer orientation. For example, a red background color of the field of view may indicate that the current transducer position and/or the current transducer orientation comprise a second deviation of the target transducer position and/or the target transducer orientation, wherein the second deviation may be larger than the first deviation. Conceivably, alternative color schemes may be used. In addition, the visualization may comprise geometrical shapes, which may differentiate between deviations regarding the current transducer position versus deviations regarding the current transducer orientation.

The visualization of the proposed user action may include a visual signal, for example an arrow which indicates a direction of a required or proposed placement adjustment. The visual signal may start at the current transducer position and lead to the target transducer position and/or start at the current transducer orientation and lead to the target transducer orientation.

The visualization may further indicate improvement possibilities as for example a change of a contact pressure.

The haptic feedback and/or the vibrotactile feedback to the user may escalate accordingly to the color code. For example the green background color may correspond to a deactivation of the haptic feedback and/or the vibrotactile feedback, the orange background color may correspond to a first intensity of the haptic feedback and/or the vibrotactile feedback and the red background color may correspond to a second intensity of the haptic feedback and/or the vibrotactile feedback, wherein the second intensity may be larger than the first intensity. The ultrasonic transducer may comprise piezoelectric actuators that produce directional vibrations and thus activate the haptic feedback and/or the vibrotactile feedback.

The visual feedback to the user via LED indicators of the ultrasonic transducer may escalate accordingly to the color code. The ultrasonic transducer may comprise LED indicators, which comprise a possibility activating different colors. In order to implement a straightforward solution, the LED indicators may comprise a similar color compared to the visualization of the field of view by the at least one display device.

An advantage of such implementations is that the user may get an unambiguous feedback, which assists to change the current transducer position and/or the current transducer orientation accordingly. Several possibilities of the transducer assisting feedback are described. A further implementation may leave a choice of the preferred transducer assisting feedback possibility to the user and thus adapt to different user types.

According to several further implementations, the current transducer position and/or the current transducer orientation is determined by the transducer monitoring system by optical tracking of the ultrasonic transducer and/or by video tracking of the ultrasonic transducer and/or by electromagnetic tracking of the ultrasonic transducer.

The ultrasonic transducer may for example comprise respective markers, which allow a camera or a camera system to recognize the current transducer position and/or the current transducer orientation via optical tracking.

The transducer monitoring system may comprise for example a 3D camera or a 3D camera system, which is configured to recognize the current transducer position and/or the current transducer orientation via video tracking.

The transducer monitoring system may comprise for example an electromagnetic field generator and the ultrasonic transducer may for example comprise a corresponding sensor, which may be configured to induce a current depending on the electromagnetic field of the electromagnetic field generator. The transducer monitoring system may recognize the current transducer position and/or the current transducer orientation via electromagnetic tracking depending on the current of the sensor.

An advantage of such implementations is that the method may assist the user precisely and in real-time as a deviation of the transducer pose may be detected quickly and accurately.

According to several further implementations, the method comprises receiving a current device position of the medical device and/or a current device orientation of the medical device from a device monitoring system and generating a device assisting feedback for assisting a user in adjusting the current device position and/or the current device orientation according to the target device path by the data processing system.

The medical device, in particular a tip of the medical device, may for example be inside the object. The user may for example manually guide the medical device and the method may assist the guidance.

An advantage of such implementations is that the user may receive a comprehensive feedback of the intervention progress by a combination of the transducer assisting feedback and the device assisting feedback. The user may be able to adapt a movement of the medical device according to the device assisting feedback.

According to several further implementations, generating the device assisting feedback comprises displaying a visualization of the current device position and/or the current device orientation by at least one display device. Additionally or alternatively, the device assisting feedback comprises displaying a visualization of a proposed further user action to adjust a placement of the medical device to reach the target device path starting from the current device position and/or the current device orientation.

The at least one display device may for example display a further overlaid view including for example the visualization of the current device position and/or the current device orientation and the visualization of the predefined imaging data.

The proposed further user action may include a further visual signal, for example a further arrow which indicates a direction of the required placement adjustment of the medical device. The further visual signal may start at the current device position and lead to a starting point of the target device path and/or start at the current device orientation and lead to a linear extension of the target device path.

An advantage of such implementations is that both the ultrasonic transducer and the medical device may be monitored by the user on the at least one display device. The user may receive direct feedback if the medical device deviates from the target device path and for example an injury of the patient may be expected. Then the method may give an indication to the user.

According to several further implementations, the at least one display device comprises an extended reality device.

The extended reality device may comprise an augmented reality device or may consist of the augmented reality device. In particular, the augmented reality device may be configured to combine parts of a real-world environment with computer-generated content. Especially the user may have a possibility to see both a real object and a three-dimensional or a two-dimensional computer-generated content simultaneously. In the present context, the computer-generated content comprises one or more of the visualizations described above.

The extended reality device may also comprise of a virtual reality device or may consist of the virtual reality device. The virtual reality device may be configured to display an image of the real object and one or more of the visualizations described above

The computer-generated content may be an image, a geometric shape, a text or another visual effect. In particular, the extended reality device may be a wearable device, which may comprise at least partially a transparent area, similar to a pair of glasses. The wearable device may be configured to show the computer-generated content in a position related to the real object and allow the user to see the real object through the transparent area. The computer-generated content may be displayed on a different layer as the real object. Especially it may be possible to move a position of the extended reality device in relation to the real object. Even during or after the movement of the position, a combined arrangement of the computer-generated content and the real object may stay unchanged.

In other embodiments, the extended reality device may comprise an extended reality headset, extended reality glasses, a smartphone, a tablet device or a camera with a display. In particular, the extended reality device may for example comprise a head-mounted display.

An advantage of such implementations is the availability of the extended reality and an ease of use for the user. The user may be able to see the patient, the transducer assisting feedback and the device assisting feedback at the same time and adapt the behavior accordingly.

According to several further implementations, the current device position and/or the current device orientation is determined by the device monitoring system by optical tracking of the medical device and/or video tracking of the medical device and/or electromagnetic tracking of the medical device.

The medical device may for example comprise further specific markers, which allow the camera or the camera system to recognize the current device position and/or the current device orientation via optical tracking.

The device monitoring system may comprise for example a further 3D camera or a further 3D camera system, which is configured to recognize the current device position and/or the current device orientation via video tracking.

The device monitoring system may comprise for example a further electromagnetic field generator and the medical device may for example comprise a further corresponding sensor, which may be configured to induce a further current depending on the further electromagnetic field of the further electromagnetic field generator. The device monitoring system may recognize the current device position and/or the current device orientation via electromagnetic tracking depending on the further current of the further sensor.

An advantage of such implementations is that the method may assist the user precisely and in real-time as a deviation of the device pose may be detected quickly and accurately.

According to several further implementations, the method comprises determining a landmark on a surface of the object by the data processing system depending on the predetermined imaging data and depending on the target region. The method further comprises generating a visualization of the landmark by the data processing system and displaying the visualization of the landmark by the at least one display device. Furthermore the method comprises generating an assisting instruction to the user by the data processing system, wherein the assisting instruction utilizes the landmark in order to guide the user to place the ultrasonic transducer at the target transducer position.

The landmark may be an identifiable and traceable position or region on the surface of the object, in particular on the skin of the patient. The landmark may for example correspond to a mid-clavicular line, a rib, an intercostal space, an acromastium or the navel.

The method comprises finding a suitable landmark depending on the target region and the predetermined imaging data. The landmark may for example be in close vicinity to the starting position of the target device path. The visualization of the landmark may be part of the transducer assisting feedback and/or the device assisting feedback.

The assisting instruction may be displayed by the at least one display device and/or made available to the user by an acoustic signal or a synthesized voice output. The position of the landmark may act as a reference position for the user to locate the target transducer position and/or the starting point of the target device path.

An advantage of such implementations is that the user assistance is increased towards an enhanced workflow also for users with a lower experience level. The user may find the correct position for the ultrasonic transducer as well as for the medical device fast and reproducible.

According to several further implementations, the medical device comprises a medical intervention needle.

The medical intervention needle may be applicable for a medical intervention and, for example, for an injection in the skin of the patient. In particular, the medical intervention needle may be applicable for percutaneous interventions, wherein the medical intervention needle may for example be a stiff needle. In particular the medical device may be an ablation needle configured for an ablation procedure.

An advantage of such implementations is that the placement of the medical intervention needle on the skin of the patient may be enhanced, and the risk of an injury of the patient is minimized.

According to several further implementations, the medical imaging data comprises or consists a computed tomography reconstruction, in particular a computed tomographic 3D reconstruction or a cone beam computed tomography reconstruction, or a magnetic resonance imaging reconstruction, in particular a 3D magnetic resonance imaging reconstruction.

An advantage of such implementations is that the computed tomography reconstruction or the magnetic resonance imaging reconstruction may offer a high degree of details and precision in order to improve the assisting for the user.

According to several further implementations, the method comprises simulating ultrasonic imaging data according to the target transducer position and/or the target transducer orientation by the data processing system depending on the predetermined imaging data. Furthermore the transducer assisting feedback is generated depending on the ultrasonic imaging data. In particular generating the transducer assisting feedback may comprise displaying a visualization of the ultrasonic imaging data by the at least one display device.

The simulation of the ultrasonic imaging data may comprise generating 2D simulation imaging data, which shall represent an expected ultrasonic imaging data at the target transducer position and/or with the target transducer orientation. The data processing unit may simulate the ultrasonic imaging data depending on the predetermined imaging data, in particular from 3D predetermined imaging data. The simulation may consider an imaging quality, which may be expected from the ultrasonic transducer. In particular a semitransparent structure, a transparent structure and a blocking structure may be considered within the simulation.

Thus, the method may comprise comparing the simulated ultrasonic imaging data with the ultrasonic imaging data generated by the ultrasonic transducer.

The at least one display device may for example display a further overlaid view including for example the visualization of the simulated ultrasonic imaging data and a visualization of the ultrasonic imaging data generated by the ultrasonic transducer. Alternatively or additionally, the transducer assisting feedback may comprise a further color code in order to indicate a correct transducer position and/or a correct transducer orientation with regard to the image comparison.

An advantage of such implementations is, that the user may be prepared for the ultrasonic imaging data and may be able to recognize quickly if the current transducer position and/or the current transducer orientation requires adaption. Additionally the user may receive a cue for a representation of the target region within the ultrasonic imaging data.

According to a further aspect of the invention, a data processing system is provided. The data processing system is configured to carry out a method according to the invention, in particular any implementation of a method according to the invention, wherein all steps of the respective method are carried out by the data processing system.

In the present disclosure, the expressions "data processing system" and "at least one data processing system" may be used interchangeably. A data processing system may in particular be understood as a data processing system, which comprises processing circuitry. The data processing system may therefore in particular process data to perform computing operations. This may also include operations to perform indexed accesses to a data structure, for example a look-up table, LUT, as well as a data processing process implemented in hardware.

In particular, the data processing system may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing system may also include one or more processors, for example one or more microprocessors, one or more central processing units, CPU, one or more graphics processing units, GPU, and/or one or more signal processors, in particular one or more digital signal processors, DSP. The data processing system may also include a physical or a virtual cluster of computers or other of said units.

In various embodiments, the data processing system includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a user assistance system for assisting a placement of an ultrasonic transducer on an object is provided. The user assistance system comprises the data processing system and/or the ultrasonic transducer and/or the transducer monitoring system and/or the device monitoring system and/or the medical device.

Further implementations of the user assistance system according to the invention follow directly from the various embodiments of the methods according to the invention and vice versa. In particular, individual features and corresponding explanations as well as advantages relating to the various implementations of the method according to the invention can be transferred analogously to corresponding implementations of the user assistance system according to the invention. In particular, the user assistance system according to the invention is designed or programmed to carry out a method according to the invention. In particular, the user assistance system according to the invention carries out a method according to the invention.

According to several implementations, the user assistance system comprises an imaging system, which is configured to generate the predetermined imaging data of the object or a part of the object.

The imaging system may for example be or comprise a medical imaging system or a computed tomography (CT) imaging system. In other embodiments, the imaging system may comprise a cone beam CT system, a fluoroscopy system, an angiography system, a C-arm system and/or a classic X-ray machine.

The imaging system may for example be or comprise a magnetic resonance imaging system.

According to a further aspect of the invention, a computer program product is provided. The computer program product comprises instructions, which, when executed by a data processing system, cause the data processing system to carry out a method according to the invention.

The instructions may be provided as program code, for example. The program code can for example be provided as binary code or assembler and/or as source code of a programming language, for example C, and/or as program script, for example Python.

The computer program product may be a computer program comprising the instructions or a computer-readable storage medium storing said computer program.

Further features and feature combinations of the invention are obtained from the figures and their description as well as the claims. In particular, further implementations of the invention may not necessarily contain all features of one of the claims. Further embodiments of the inventions may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically aspects of an intervention, which is assisted using an exemplary implementation of a method for assisting a placement of an ultrasonic transducer according to the invention;
- FIG 2: shows schematically aspects of an intervention, which is assisted using a further exemplary implementation of a method for assisting a placement of an ultrasonic transducer according to the invention;
- FIG 3: shows schematically an exemplary implementation of a user assistance system according to the invention;
- FIG 4: shows schematically a transducer assisting feedback in a further exemplary implementation of a method according to the invention;
- FIG 5: shows schematically a transducer assisting feedback in a further exemplary implementation of a method according to the invention;
- FIG 6: shows a schematic flow diagram of an exemplary implementation of a method for an assisted ultrasonic examination according to the invention; and
- FIG 7: shows schematically an artificial neural network; and

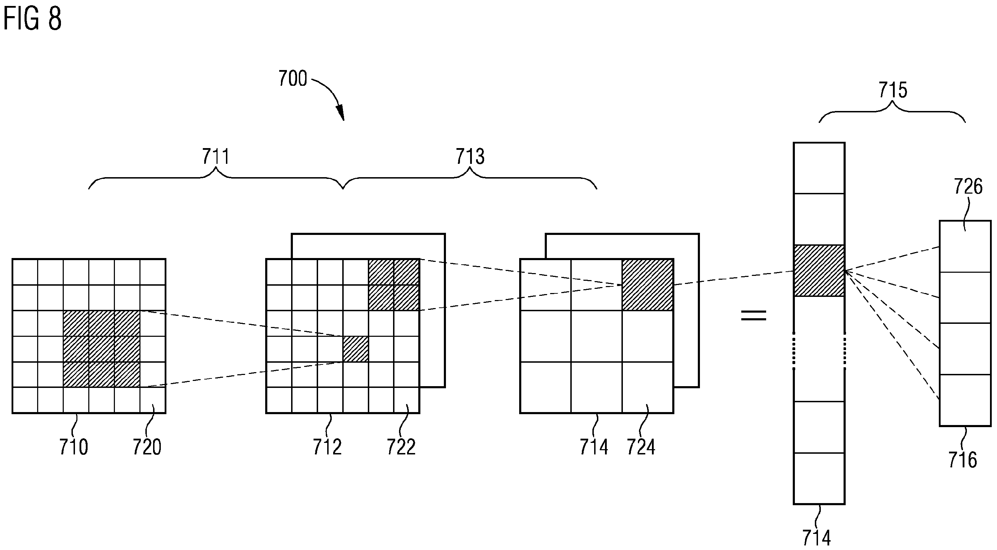

- FIG 8: shows schematically a convolutional neural network.

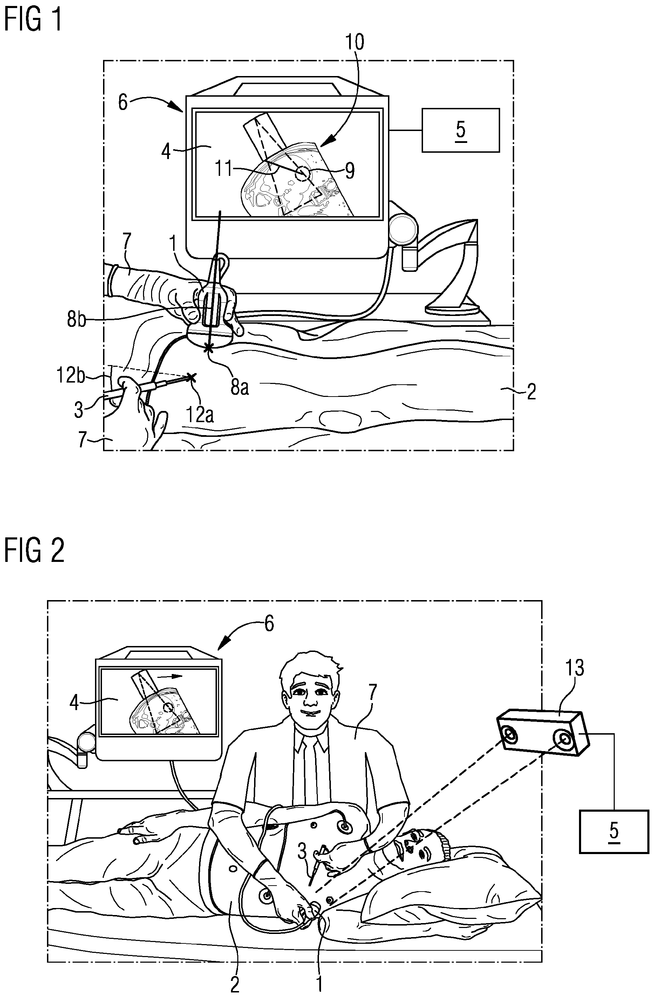

FIG 1 schematically shows aspects of an intervention, which is assisted using an exemplary implementation of a method for assisting a placement of an ultrasonic transducer according to the invention. An ultrasonic transducer 1 may be placed on an object 2 to monitor a medical device 3, for example an intervention needle.

A data processing system 5 is configured to receive a target region 9 and predetermined imaging data 10 and to determine a target device path 11 for the medical device 3 to the target region 9 and an acoustic window for the target device path 11 depending on the predetermined imaging data 10.

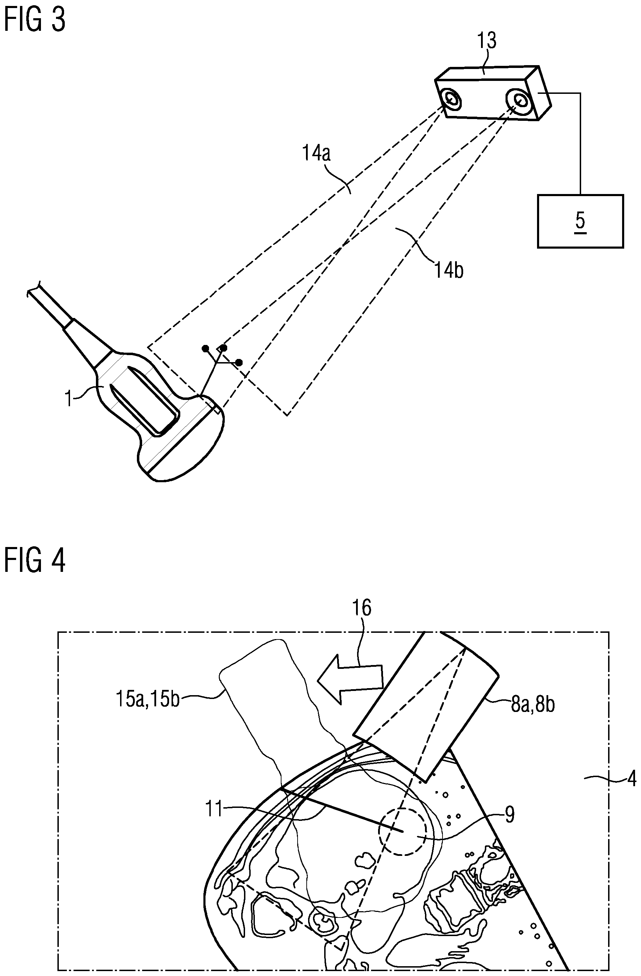

The data processing system 5 is furthermore configured to determine a target transducer position 15a (see FIG 4) and/or a target transducer orientation 15b (see FIG 4) depending on the acoustic window and receiving a current transducer position 8a and/or a current transducer orientation 8b of the ultrasonic transducer 1. A transducer assisting feedback 4 may be generated by the data processing system 5 and displayed on at least one display device 6. Via the transducer assisting feedback 4, a user 7 may receive feedback on the current transducer position 8a and/or the current transducer orientation 8b depending on the target transducer position and/or the target transducer orientation.

In the implementation example shown in FIG 1 the transducer assisting feedback 4 comprises a visualization of the target device path 11, a visualization of the predetermined imaging data 10, a visualization of the target region 9, a visualization of the ultrasonic transducer 1, a visualization of the field of view of the ultrasonic transducer 1.

Furthermore the data processing system 5 may be configured to receive a current device position 12a of the medical device 3 and/or a current device orientation 12b of the medical device and to generate a device assisting feedback for assisting a user 7 in adjusting the current device position 12a and/or the current device orientation according to the target device path 11.

A medical intervention, which is assisted by one of the implementations of the method according to the invention, may start with the user 7 to receive the target device path 11 and the target transducer position 15a and/or the target transducer orientation 15b for example displayed on the display device 6. The user 7 may be an operator for the medical intervention, especially a physician, a physician assistant or an interventional radiologist (IR). The medical device 3 may for example be an intervention needle or an ablation needle. The target region 9 may for example be a tumor or a lesion and the object 2 may be a patient. The goal of the medical intervention may be enable physical contact between the target region 9 and the medical device 3 for example in order to initiate a treatment or to take a specimen. The method may assist the operator to establish the physical contact. The predetermined imaging data 10 may have been generated by a computed tomography (CT) imaging system and a visualization of the predetermined imaging data 10 may be displayed on the display device 6. The method may additionally include a further display device, which is configured to display ultrasonic imaging data, which are generated by a signal from the ultrasonic transducer 1. The method may assist the operator to interpret the ultrasonic imaging data with a help of the transducer assisting feedback 4.

The operator may place the intervention needle on the skin of the patient at the current device position 12a, with a goal to place the intervention needle at a starting point of the target device path 11. Next to that the operator may place the ultrasonic transducer 1 on the skin of the patient at the current transducer position 8a, with a goal to place the ultrasonic transducer 1 at the target transducer position 15a. The transducer assisting feedback 4 may assist the operator to inject and/or to progress the intervention needle by continuously finding a suitable current transducer position 8a and/or a suitable current transducer orientation 8b, which allows to supervise the injection and/or the progress of the intervention needle.

In some implementations, the target device path 11 and the acoustic window are determined jointly. In particular, the target device path 11 is determined such that ultrasonic imaging of the medical device 3 along the target device path 11 is possible according to the acoustic window.

This may, for example be achieved by an alternating optimization approach or by applying an MLM, which has been trained according, to input data, which depends on or comprises or consists of the predetermined imaging data 10 and the target region 9.

FIG 2 shows schematically aspects of a further exemplary implementation. All or a part of the features described for the implementation shown in FIG 1 may apply to the implementation shown in FIG 2 if not described otherwise.

The current transducer position 8a and/or the current transducer orientation 8b may be determined by a transducer monitoring system 13 for example by optical tracking, in particular by using a camera, for example a 3D camera system. The data processing system 5 may be configured to receive a signal from the transducer monitoring system 13.

FIG 3 shows a further implementation of the transducer monitoring system 13, which is configured to determine the current transducer position 8a and/or the current transducer orientation 8b by optical tracking. The optical tracking may comprise a stereo camera and/or a 3D camera system and/or a light emission unit and/or optical markers that act as reflectors to a light of the light emission unit. Using triangulation, a current position of the optical markers and/or a current orientation of the optical markers and correspondingly the current position of the ultrasonic transducer and/or the current orientation of the ultrasonic transducer may be determined. The transducer monitoring system 13 may comprise a first camera to generate an image of the ultrasonic transducer 1 from a first field of view 14a. The transducer monitoring system 13 may comprise a second camera to generate a further image of the ultrasonic transducer 1 from a second field of view 14b. The transducer monitoring system 13 or the data processing system 5 may be configured to process the image and the further image in order to determine the current transducer position 8a and/or the current transducer orientation 8b.

The device monitoring system (not shown) may comprise all or a part of the features described for the transducer monitoring system 13, whereas the device monitoring system is configured to determine the current device position 12a and/or the current device orientation 12b. The device monitoring system and the transducer monitoring system 13 may be integrated in a single system or may be provided separately.

FIG 4 shows a further implementation of the transducer assisting feedback 4, which may be displayed on the at least one display device 6. The transducer assisting feedback 4 may comprise the current transducer position 8a and/or the current transducer orientation 8b and/or the target transducer position 15a and/or the target transducer orientation 15b. FIG 4 shows an example of a deviation of the current transducer position 8a from the target transducer position 15a and a further deviation of the current transducer orientation 8b from the target transducer orientation 15b. The user 7 may receive a proposed user action 16 in order to assist the placement of the ultrasonic transducer 1. The deviation and/or the further deviation in FIG 4 are shown as a lateral deviation. The proposed user action 16 is visualized as an arrow to indicate a required user action 16. In particular, the arrow indicates a direction in which the ultrasonic transducer 1 should be moved according to the proposed user action 16.

The transducer assisting feedback 4 may comprise an overlaid view of the above-described visualizations with a background layer representing the predetermined imaging data 10 in order to assist the placement of the ultrasonic transducer 1. The background layer in FIG 4 shows a lateral sectional view of the object 2, in particular of the patient.

The method may comprise generating a device assisting feedback (not shown). The device assisting feedback may comprise all or a part of the features described for the transducer assisting feedback 4 or analogous features, respectively, whereas the device assisting feedback comprises displaying a visualization of the current device position 12a and/or the current device orientation 12b by the at least one display device. Additionally the device assisting feedback may comprise displaying a visualization of a proposed further user action 16 to adjust a placement of the medical device to reach the target device path starting from the current device position and/or the current device orientation.

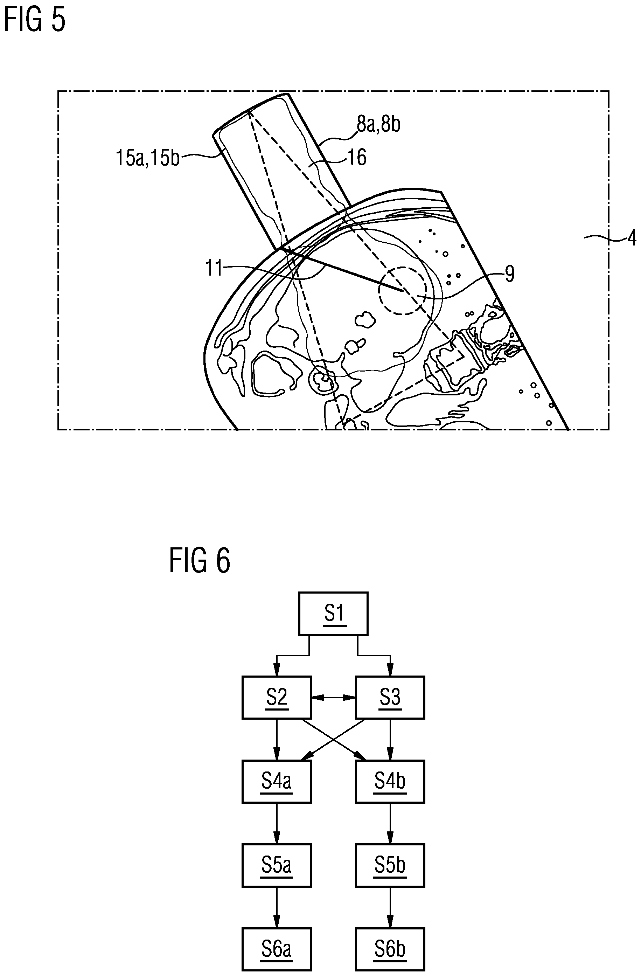

FIG 5 shows a further implementation of the transducer assisting feedback 4, which may be displayed on the at least one display device 6. The features of the transducer assisting feedback 4 in FIG 5 may be similar to FIG 4 unless otherwise stated. In comparison to FIG 4, the deviation and/or the further deviation are shown as a caudal/cranial deviation. Thus, the proposed user action 16 may be a geometrical shape in the lateral sectional view, in other words the proposed user action 16 may be perpendicular to the lateral sectional view.

FIG 6 shows a schematic flow diagram of an exemplary implementation of a method for assisting the placement of the ultrasonic transducer 1 on the object 2 to monitor the medical device 3 according to the invention.

The target region 9 inside the object 2 and predetermined imaging data 10 of the object 2 or of a part of the object 2 are received S1 by the data processing system 5. The target device path 11 for the medical device 3 to the target region 9 and the acoustic window for the target device path 11 are determined S2, S3 by the data processing system 5 depending on the predetermined imaging data 10. The method further comprises determining S4a the target transducer position 15a for the ultrasonic transducer 1 by the data processing system 5 depending on the acoustic window, receiving S5a the current transducer position 8a of the ultrasonic transducer 1 from the transducer monitoring system 13 by the data processing system 5 and generating S6a the transducer assisting feedback 4 for assisting the user 7 in placing the ultrasonic transducer 1 on the object 2 depending on the target transducer position 15a and the current transducer position 8a by the data processing system 5. The method further comprises determining S4b the target transducer orientation 15b for the ultrasonic transducer 1 by the data processing system 5 depending on the acoustic window, receiving S5b the current transducer orientation 8b of the ultrasonic transducer 1 from the transducer monitoring system 13 by the data processing system 5 and generating S6b the transducer assisting feedback 4 for assisting the user 7 in placing the ultrasonic transducer 1 on the object 2 depending on the target transducer orientation 15b and the current transducer orientation 8b by the data processing system 5.

In some embodiments, a software solution is proposed that enables to assist minimal invasive interventions, for example during percutaneous and laparoscopic procedures, based on ultrasonic guidance with minimum ultrasonic imaging experience. The approach may build on top of an algorithm, which is available for ablation planning, for example. In particular, the approach may build on a planning algorithm, which enables automated ablation planning for liver tumor ablations.

The planning algorithm may calculate an optimal ablation plan for a liver tumor based on risk structure segmentation (e.g., skeleton, rib cartilages, organs) proposing a safe target device path 11, which may be a needle trajectory that allows for full margin coverage (A0 ablation).

Compared to live CT guidance of interventions, ultrasonic guidance adds a need to have an ultrasonic line of sight. As ultrasonic may not penetrate bone or gas, and the liver is surrounded by ribs circumferentially and gas filled organs above and below, there is a limited amount of imaging windows which can see a given target region 9, for example a tumor, and an entire needle trajectory which does not go across any critical structure such as a vessel or a neighbor organ.

Finding the entire needle trajectory manually may require a user 7, which is trained and experienced, some embodiments of the current invention extend the algorithm to find those imaging windows, where a combination of factors is present, reducing a workload for the operator, and may help to assess whether the intervention is feasible with ultrasonic guidance in the first place.

One potential workflow may be described as follows:
1. On the day of the intervention or before the day of the intervention, a contrast enhanced predetermined imaging data, for example via a CT imaging system, of the patient is taken.
2. The operator or the IR may plan an ablation plan for the patient according to the method including determining the target device path 11 and the acoustic window by the data processing system 5.
3. The method comprises confining a solution space to anatomical imaging windows where ultrasonic imaging is possible and, for example, optimal for the patient. The anatomical imaging windows can be found based on an existing organ segmentation. The method may use rib, cartilage, and organ segmentation masks to determine an optimal acoustical window for the ultrasonic transducer 1, where the tumor can be seen optimally.
4. In a next step, the target transducer position 15a and/or the target transducer orientation 15b on the patient, which may correspond to the anatomical imaging window will be calculated and visualized by the display device 6. Using optical tracking, the current transducer position 8a and/or the current transducer orientation 8b may be tracked and correlated to the target transducer position 15a and/or the target transducer orientation 15b. The IR can be guided to the anatomical imaging window with, e.g., a color code where green is the target transducer position 15a and/or the target transducer orientation 15b.

Optimal imaging windows can be calculated deterministically by, e.g., raytracing algorithms where anatomical windows are calculated based on the segmentation masks and optimization algorithms are used to find the optimal imaging window.

Alternatively or additionally, optimal imaging windows can be found using reinforcement learning where an agent learns to find the optimal imaging window. Alternatively or additionally, deep learning can be used to train a neural network with optimal imaging windows and find the optimal imaging window.

Another embodiment relates to EM tracking instead of optical tracking to find the current transducer position 15a and/or the current transducer orientation 15b. Another embodiment relates to using a 3D camera instead of optical tracking/EM to find the current transducer position 15a and/or the current transducer orientation 15b. In this case, no markers are needed to track the ultrasonic transducer 1 and the medical device 3. Instead, a 3D model of the ultrasonic transducer 1 and the medical device 3 is generated, which will be used by a camera to do a 3D tracking with the 3D camera.

The method may translate planned procedural positions into standard anatomical landmarks and may provide real-time, multimodal feedback to the IR. By integrating algorithms for anatomical mapping, visualization tools, and tracking technologies, the system enhances the accuracy and efficiency of the placement of the intervention needle and ablations.

The method may automatically identify standard anatomical landmarks relevant to the procedure (e.g., mid-clavicular line, ribs, intercostal spaces). The method may convert the target transducer position 15a and/or the target transducer orientation 15b into actionable instructions using clinical terminology familiar to the IR (for example: "Place the ultrasonic transducer 1 two centimeters ventral to the mid-clavicular line, between the 7th and 8th ribs, aligned with the intercostal space at an 8-degree cranial angle."). The method may determine the optimal transducer angle relative to anatomical planes, aiding in precise needle guidance.

The display device 6 may provide a 3D visualization of the patient's anatomy with overlaid guidance markers indicating the target transducer position 15a and/or the target transducer orientation 15b. The 3D visualization may be modifiable to better understand spatial relationships. The display device 6 may display real-time positioning cues on the ultrasonic machine's monitor or a separate display. The display device 6 may display which anatomical structures should be visible in the ultrasonic imaging data when correctly positioned. The display device 6 may project guidance information onto AR glasses or tablet screens, overlaying instructions directly onto the patient's body.

The method may utilize optical, electromagnetic, or video-based tracking systems attached to the ultrasonic transducer 1. The method may continuously monitor the current transducer position 8a and/or the current transducer orientation 8b in relation to the patient's anatomy. The ultrasonic transducer 1 and/or the medical device 3 may incorporate piezoelectric actuators that produce directional vibrations, guiding the hand of the IR subtly toward the target transducer position 15a and/or the target transducer orientation 15b. The ultrasonic transducer 1 may comprise color-coded LEDs to provide immediate visual cues (e.g., green for correct alignment, red for adjustment needed). The method may compare live ultrasonic imaging data with simulated images derived from the predetermined imaging data 10, confirming the visibility of key structures. The method may comprise notifying the IR if the expected anatomical features are not visible, suggesting adjustments.

The method may comprise adjusting the predetermined imaging data to account for organ displacement due to pneumoperitoneum, providing accurate models for laparoscopic conditions. The method may comprise simulating the inflated abdominal cavity to reflect true intraoperative anatomy. The method may comprise using laparoscopic camera feeds to track the current transducer position 8a and/or the current transducer orientation 8b on the organ surface. The method may project guidance markers and instructions directly onto the laparoscopic video, aiding in precise transducer placement. The method may supply exact skin entry points and the target transducer position 15a using standardized anatomical reference points. The method may assists in planning and placing laparoscopic trocars based on common surgical landmarks.

The IR may select target lesions and plan the target device path 11 or the needle trajectory based on the predetermined imaging data 10. The method may comprise generating a comprehensive guidance plan, translating this into anatomical instructions.

The IR may need to follow the steps:
- attach tracking sensors to the ultrasonic transducer 1;
- initialize the data processing system 5 with patient-specific data and calibrate as necessary;
- follow textual and visual instructions to position the ultrasonic transducer 1;
- real-time tracking provides continuous feedback, with the data processing system 5 adjusting guidance as needed;
- verification steps confirm the visibility of key anatomical structures and alignment with the target device path 11.

As mentioned above, an MLM may be used in some implementations to determine the target device path 11 and the acoustic window.

The MLM may for example be an artificial neural network, ANN. FIG 6 displays an embodiment of an ANN 800, which is for example designed as an MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 6, the edge 840 is a directed connection from the node 820 to the node 823, and the edge 842 is a directed connection from the node 830 to the node 832. An edge 840, ..., 842 from a first node 820, ..., 832 to a second node 820, ..., 832 is also denoted as ingoing edge for the second node 820, ..., 832 and as outgoing edge for the first node 820, ..., 832.

In this example, the nodes 820, ..., 832 of the artificial neural network 800 can be arranged in layers 810, ..., 813, wherein the layers can comprise an intrinsic order introduced by the edges 840, ..., 842 between the nodes 820, ..., 832. In particular, edges 840, ..., 842 can exist only between neighboring layers of nodes. In the displayed example, there is an input layer 810 comprising only nodes 820, ..., 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ..., 832 of the artificial neural network 800. Here, x(n)i denotes the value of the i-th node 820, ..., 832 of the n-th layer 810, ..., 813. The values of the nodes 820, ..., 822 of the input layer 810 are equivalent to the input values of the artificial neural network 800. The values of the nodes 831, 832 of the output layer 813 are equivalent to the output value of the artificial neural network 800.

**Furthermore,** each edge 840, ..., 842 can comprise a weight being a real number. In particular, the weight is a real number within the interval [-1, 1] or within the interval [0, 1]. Here, w^{(m,n)}_{i,j} denotes the weight of the edge between the i-th node 820, ..., 832 of the m-th layer 810, ..., 813 and the j-th node 820, ..., 832 of the n-th layer 810, ..., 813. Furthermore, the abbreviation w⁽ⁿ⁾_{i,j} is defined for the weight w^{(n,n+1)}_{i,j}. In particular, to calculate the output values of the neural network 800, the input values are propagated through the neural network 800. In particular, the values of the nodes 820, ..., 832 of the (n+1)-th layer 810, ..., 813 can be calculated based on the values of the nodes 820, ..., 832 of the n-th layer 810, ..., 813 by ${x}_{j}^{\left(n+1\right)} = f \left({\sum }_{i} {x}_{i}^{\left(n\right)} {w}_{i , j}^{\left(n\right)}\right) .$

**Herein, the** function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is for example used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values w^{(m,n)}_{i,j} for the edges, the neural network 800 has to be trained using training data. In particular, training data comprises training input data and training output data (denoted as tᵢ). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to ${w}_{i , j}^{′ \left(n\right)} = {w}_{i , j}^{\left(n\right)} - γ {δ}_{j}^{\left(n\right)} {x}_{i}^{\left(n\right)} ,$ wherein γ is a predefined learning rate, and the numbers δ⁽ⁿ⁾ⱼ can be recursively calculated as ${δ}_{j}^{\left(n\right)} = \left({\sum }_{k} {δ}_{k}^{\left(n+1\right)} {w}_{j , k}^{\left(n+1\right)}\right) f ′ \left({x}_{i}^{\left(n\right)}{w}_{i , j}^{\left(n\right)}\right)$ based on δ⁽ⁿ⁺¹⁾ⱼ, if the (n+1)-th layer is not the output layer 813, and ${δ}_{j}^{\left(n\right)} = \left({x}_{j}^{\left(n+1\right)}-{t}_{j}^{\left(n+1\right)}\right) f ′ \left({x}_{i}^{\left(n\right)}{w}_{i , j}^{\left(n\right)}\right) ,$ if the (n+1)-th layer is the output layer 813, wherein f' is the first derivative of the activation function, and t⁽ⁿ⁺¹⁾ⱼ is the comparison training value for the j-th node of the output layer 813.

The MLM may for example be a convolutional neural network, CNN. A convolutional neural network, CNN, is an ANN that uses a convolution operation instead of general matrix multiplication in at least one of its layers. These layers are denoted as convolutional layers. In particular, a convolutional layer performs a dot product of one or more convolution kernels with the convolutional layer's input data, wherein the entries of the one or more convolution kernel are parameters or weights that may be adapted by training. In particular, one can use the Frobenius inner product and the ReLU activation function. A convolutional neural network can comprise additional layers, for example pooling layers, fully connected layers, and/or normalization layers.

By using convolutional neural networks, the input can be processed in a very efficient way because a convolution operation based on different kernels can extract various image features so that by adapting the weights of the convolution kernel the relevant image features can be found during training. Furthermore, based on the weight-sharing in the convolutional kernels fewer parameters need to be trained, which prevents overfitting in the training phase and allows to have faster training or more layers in the network, improving the performance of the network.

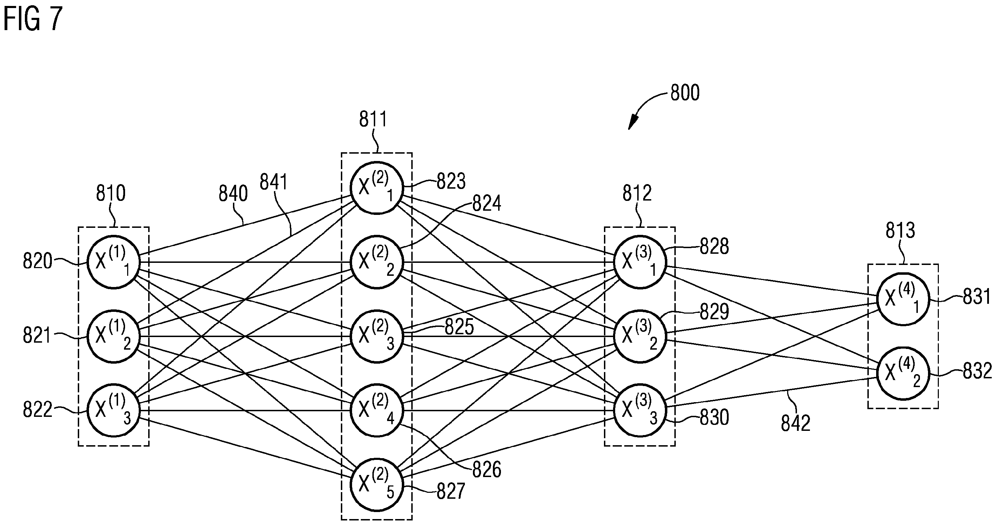

FIG 7 displays an exemplary embodiment of a convolutional neural network 700. In the displayed embodiment, the convolutional neural network 700 comprises an input node layer 710, a convolutional layer 711, a pooling layer 713, a fully connected layer 714 and an output node layer 716, as well as hidden node layers 712, 714. Alternatively, the convolutional neural network 200 can comprise several convolutional layers 711, several pooling layers 713 and/or several fully connected layers 715, as well as other types of layers. The order of the layers can be chosen arbitrarily, usually fully connected layers 715 are used as the last layers before the output layer 716.

In particular, within a convolutional neural network 700 nodes 720, 722, 724 of a node layer 710, 712, 714 can be considered to be arranged as a d-dimensional matrix or as a d-dimensional image. In particular, in the two-dimensional case the value of the node 720, 722, 724 indexed with i and j in the n-th node layer 710, 712, 714 can be denoted as x(n)[i, j]. However, the arrangement of the nodes 720, 722, 724 of one node layer 710, 712, 714 does not have an effect on the calculations executed within the convolutional neural network 700 as such, since these are given solely by the structure and the weights of the edges.

A convolutional layer 711 is a connection layer between an anterior node layer 710 with node values x(n-1) and a posterior node layer 712 with node values x(n). In particular, a convolutional layer 711 is characterized by the structure and the weights of the incoming edges forming a convolution operation based on a certain number of kernels. In particular, the structure and the weights of the edges of the convolutional layer 711 are chosen such that the values x(n) of the nodes 722 of the posterior node layer 712 are calculated as a convolution x(n) = K * x(n-1) based on the values x(n-1) of the nodes 720 anterior node layer 710, where the convolution * is defined in the two-dimensional case as ${x}^{\left(n\right)} \left[i, j\right] = \left(K∗{x}^{\left(n-1\right)}\right) \left[i, j\right] = {\sum }_{i ′} {\sum }_{j ′} K \left[i′,j′\right] ⋅ {x}^{\left(n-1\right)} \left[i-i′,j-j′\right] .$

Herein, the kernel K is a d-dimensional matrix, in the present example a two-dimensional matrix, which is usually small compared to the number of nodes 720, 722, for example a 3x3 matrix, or a 5x5 matrix. In particular, this implies that the weights of the edges in the convolution layer 711 are not independent, but chosen such that they produce said convolution equation. In particular, for a kernel being a 3x3 matrix, there are only 9 independent weights, each entry of the kernel matrix corresponding to one independent weight, irrespectively of the number of nodes 720, 722 in the anterior node layer 710 and the posterior node layer 712.

In general, convolutional neural networks 700 use node layers 710, 712, 714 with a plurality of channels, in particular, due to the use of a plurality of kernels in convolutional layers 711. In those cases, the node layers can be considered as (d+1)-dimensional matrices, the first dimension indexing the channels. The action of a convolutional layer 711 is then in a two-dimensional example defined as ${x}_{b}^{\left(n\right)} \left[i, j\right] = {\sum }_{a} \left({K}_{a , b}∗{x}_{a}^{\left(n-1\right)}\left[i, j\right]={\sum }_{a} {\sum }_{i ′} {\sum }_{j ′} {K}_{a , b} \left[i′,j′\right] ⋅ {x}_{a}^{\left(n-1\right)} \left[i-i′,j-j′\right] .\right.$ wherein ${x}_{a}^{\left(n\right)}$ corresponds to the a-th channel of the anterior node layer 710, ${x}_{b}^{\left(n\right)}$ corresponds to the b-th channel of the posterior node layer 712 and *K_{a,b}* corresponds to one of the kernels. If a convolutional layer 711 acts on an anterior node layer 710 with A channels and outputs a posterior node layer 712 with B channels, there are A·B independent d-dimensional kernels *K_{a,b}.*

In general, in convolutional neural networks 700 activation functions may be used. In this embodiment, ReLU (rectified linear unit) is used, with R(z) = max(0, z), so that the action of the convolutional layer 711 in the two-dimensional example is ${x}_{b}^{\left(n\right)} \left[i, j\right] = R \left({\sum }_{a} {K}_{a , b} ∗ {x}_{a}^{\left(n-1\right)} \left[i, j\right]\right) = R \left({\sum }_{a} {\sum }_{i ′} {\sum }_{j ′} {K}_{a , b} \left[i′,j′\right] ⋅ {x}_{a}^{\left(n-1\right)} \left[i-i′,j-j′\right]\right) .$

It is also possible to use other activation functions, for example ELU (exponential linear unit), LeakyReLU, Sigmoid, Tanh or Softmax.

In the displayed embodiment, the input layer 710 comprises 36 nodes 720, arranged as a two-dimensional 6x6 matrix. The first hidden node layer 712 comprises 72 nodes 722, arranged as two two-dimensional 6x6 matrices, each of the two matrices being the result of a convolution of the values of the input layer with a 3x3 kernel within the convolutional layer 711. Equivalently, the nodes 722 of the first hidden node layer 712 can be interpreted as arranged as a three-dimensional 2x6x6 matrix, wherein the first dimension correspond to the channel dimension.

An advantage of using convolutional layers 711 is that spatially local correlation of the input data can exploited by enforcing a local connectivity pattern between nodes of adjacent layers, in particular by each node being connected to only a small region of the nodes of the preceding layer.

A pooling layer 713 is a connection layer between an anterior node layer 712 with node values x(n-1) and a posterior node layer 714 with node values x(n). In particular, a pooling layer 713 can be characterized by the structure and the weights of the edges and the activation function forming a pooling operation based on a non-linear pooling function f. For example, in the two-dimensional case the values x(n) of the nodes 724 of the posterior node layer 714 can be calculated based on the values x(n-1) of the nodes 722 of the anterior node layer 712 as ${x}_{b}^{\left(n\right)} \left[i, j\right] = f \left({x}_{b}^{\left(n-1\right)}\left[{id}_{1}, {jd}_{2}\right],…, {x}_{b}^{\left(n-1\right)}\left[\left(i+1\right){d}_{1}-1,\left(j+1\right){d}_{2}-1\right]\right) .$

In other words, by using a pooling layer 713, the number of nodes 722, 724 can be reduced by re-placing a number d1.d2 of neighboring nodes 722 in the anterior node layer 712 with a single node 722 in the posterior node layer 714 being calculated as a function of the values of said number of neighboring nodes. In particular, the pooling function f can be the max-function, the average or the L2-Norm. In particular, for a pooling layer 713 the weights of the incoming edges are fixed and are not modified by training.

The advantage of using a pooling layer 713 is that the number of nodes 722, 724 and the number of parameters is reduced. This leads to the amount of computation in the network being reduced and to a control of overfitting.

In the displayed embodiment, the pooling layer 713 is a max-pooling layer, replacing four neighboring nodes with only one node, the value being the maximum of the values of the four neighboring nodes. The max-pooling is applied to each d-dimensional matrix of the previous layer. In this embodiment, the max-pooling is applied to each of the two two-dimensional matrices, reducing the number of nodes from 72 to 18.

In general, the last layers of a convolutional neural network 700 may be fully connected layers 715. A fully connected layer 715 is a connection layer between an anterior node layer 714 and a posterior node layer 716. A fully connected layer 713 can be characterized by the fact that a majority, in particular, all edges between nodes 714 of the anterior node layer 714 and the nodes 716 of the posterior node layer are present, and wherein the weight of each of these edges can be adjusted individually.

In this embodiment, the nodes 724 of the anterior node layer 714 of the fully connected layer 715 are displayed both as two-dimensional matrices, and additionally as non-related nodes, indicated as a line of nodes, wherein the number of nodes was reduced for a better presentability. This operation is also denoted as flattening. In this embodiment, the number of nodes 726 in the posterior node layer 716 of the fully connected layer 715 smaller than the number of nodes 724 in the anterior node layer 714. Alternatively, the number of nodes 726 can be equal or larger.

Furthermore, in this embodiment the Softmax activation function is used within the fully connected layer 715. By applying the Softmax function, the sum the values of all nodes 726 of the output layer 716 is 1, and all values of all nodes 726 of the output layer 716 are real numbers between 0 and 1. In particular, if using the convolutional neural network 700 for categorizing input data, the values of the output layer 716 can be interpreted as the probability of the input data falling into one of the different categories.

In particular, convolutional neural networks 700 can be trained based on the backpropagation algorithm. For preventing overfitting, methods of regularization can be used, for example dropout of nodes 720, ..., 724, stochastic pooling, use of artificial data, weight decay based on the L1 or the L2 norm, or max norm constraints.

Independent of the grammatical term usage, individuals with male, female or other gender identities are included within the term.

## Claims

1. Method for assisting a placement of an ultrasonic transducer (1) on an object (2) to monitor a medical device (3), wherein a target region (9) inside the object (2) and predetermined imaging data (10) of the object (2) or of a part of the object (2) are received by a data processing system (5), and wherein a target device path (11) for the medical device (3) to the target region (9) and an acoustic window for the target device path (11) are determined by the data processing system (5) depending on the predetermined imaging data (10), wherein the method further comprises
- determining a target transducer position (15a) for the ultrasonic transducer (1) by the data processing system (5) depending on the acoustic window, receiving a current transducer position (8a) of the ultrasonic transducer (1) from a transducer monitoring system (13) by the data processing system (5) and generating a transducer assisting feedback (4) for assisting a user (7) in placing the ultrasonic transducer (1) on the object (2) depending on the target transducer position (15a) and the current transducer position (8a) by the data processing system (5); and/or
- determining a target transducer orientation (15b) for the ultrasonic transducer (1) by the data processing system (5) depending on the acoustic window, receiving a current transducer orientation (8b) of the ultrasonic transducer (1) from the transducer monitoring system (13) by the data processing system (5) and generating the transducer assisting feedback (4) for assisting the user (7) in placing the ultrasonic transducer (1) on the object (2) depending on the target transducer orientation (15b) and the current transducer orientation (8b) by the data processing system (5).

2. Method according to claim 1, wherein the determination of the target device path (11) and the acoustic window is carried out jointly, wherein the target device path (11) is determined such that ultrasonic imaging of the medical device (3) along the target device path (11) is possible according to the acoustic window.

3. Method according to one of the previous claims, wherein the target device path (11) and the acoustic window are determined by applying a machine learning model to input data, which depends on the predetermined imaging data (10).

4. Method according to one of the previous claims, wherein generating the transducer assisting feedback (4) comprises
- displaying a visualization of the acoustic window by at least one display device (6); and/or
- displaying a visualization of the target device path (11) by the at least one display device (6); and/or
- comparing the target transducer position (15a) with the current transducer position (8a) and/or comparing the target transducer orientation (15b) with the current transducer orientation (8b) by the data processing system (5), generating a visualization of a field of view of the ultrasonic transducer (1) for the current transducer position (8a) and/or the current transducer orientation (8b) depending on a result of the comparison by the data processing system (5), and displaying the visualization of the field of view by the at least one display device (6); and/or
- displaying a visualization of a proposed user action (16) to adjust the placement of the ultrasonic transducer (1) to reach the target transducer position (15a) starting from the current transducer position (8a) and/or to reach the target transducer orientation (15b) starting from the current transducer orientation (8b);
- generating a haptic feedback and/or vibrotactile feedback to the user (7) depending on the result of the comparison by the ultrasonic transducer (1); and/or
- generating a visual feedback to the user (7) via at least one light source of the ultrasonic transducer (1) depending on the result of the comparison; and/or
- generating a visualization of a further field of view of the ultrasonic transducer (1) for the target transducer position (15a) and/or the target transducer orientation (15b), and displaying the visualization of the further field of view by the at least one display device (6).

5. Method according to one of the previous claims, wherein the current transducer position (8a) and/or the current transducer orientation (8b) is determined by the transducer monitoring system (13) by optical tracking of the ultrasonic transducer (1) and/or by video tracking of the ultrasonic transducer (1) and/or by electromagnetic tracking of the ultrasonic transducer (1).

6. Method according to one of the previous claims, wherein the method comprises receiving a current device position (12a) of the medical device (3) and/or a current device orientation (12b) of the medical device (3) from a device monitoring system and generating a device assisting feedback for assisting the user (7) in adjusting the current device position (12a) and/or the current device orientation (12b) according to the target device path (11) by the data processing system (5).

7. Method according to claim 6, wherein generating the device assisting feedback comprises
- displaying a visualization of the current device position (12a) and/or the current device orientation (12b) by at least one display device (6); and/or
- displaying a visualization of a proposed further user action (16) to adjust a placement of the medical device (3) to reach the target device path (11) starting from the current device position (12a) and/or the current device orientation (12b).

8. Method according to claim 4 or claim 7, wherein the at least one display device (6) comprises an extended reality device.

9. Method according to one of the claims 6 or claim 7, wherein the current device position (12a) and/or the current device orientation (12b) is determined by the device monitoring system by optical tracking of the medical device (3) and/or video tracking of the medical device (3) and/or electromagnetic tracking of the medical device (3).

10. Method according to one of the previous claims, wherein the method comprises
- determining a landmark on a surface of the object (2) by the data processing system (5) depending on the predetermined imaging data (10) and depending on the target region (9);
- generating a visualization of the landmark by the data processing system (5);
- displaying the visualization of the landmark by the at least one display device (6); and
- generating an assisting instruction to the user (7) by the data processing system (5), wherein the assisting instruction utilizes the landmark in order to guide the user (7) to place the ultrasonic transducer (1) at the target transducer position (15a).

11. Method according to one of the previous claims, wherein the medical imaging data (10) comprises or consists of a computed tomography reconstruction or a magnetic resonance imaging reconstruction.

12. Method according to one of the previous claims, wherein
- the method comprises simulating ultrasonic imaging data (10) according to the target transducer position (15a) and/or the target transducer orientation (15b) by the data processing system (5) depending on the predetermined imaging data (10); and
- the transducer assisting feedback (4) is generated depending on the ultrasonic imaging data (10).

13. Data processing system (5), which is configured to carry out a method according to claim 1.

14. User assistance system for assisting a placement of an ultrasonic transducer (1) on an object (2) comprising the data processing system (5) according to claim 13, the ultrasonic transducer (1) and/or the transducer monitoring system (13) and/or the medical device.

15. Computer program product comprising instructions, which, when executed by a data processing system (5), cause the data processing system (5) to carry out a method according to claim 1.
